(19) Europäisches Patentamt
European Patent Office
Office européen des brevets

(11) Veröffentlichungsnummer: **0 334 148 A2**

(12) **EUROPÄISCHE PATENTANMELDUNG**

(21) Anmeldenummer: **89104382.0**

(22) Anmeldetag: **13.03.89**

(51) Int. Cl.⁴: **C07D 471/04 , A01N 43/90 , C07D 215/40 , C07D 215/38 , //(C07D471/04,221:00,221:00)**

(30) Priorität: **23.03.88 DE 3809776**

(43) Veröffentlichungstag der Anmeldung: **27.09.89 Patentblatt 89/39**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Heinemann, Ulrich, Dr.**
**Feldstrasse 18**
**D-5653 Leichlingen 1(DE)**
Erfinder: **Brandes, Wilhelm, Dr.**
**Eichendorffstrasse 3**
**D-5653 Leichlingen 1(DE)**

(54) **Hetaryl-1,10-phenanthrolin-Derivate.**

(57) Hetaryl-1,10-phenanthrolin-Derivate der Formel (I)

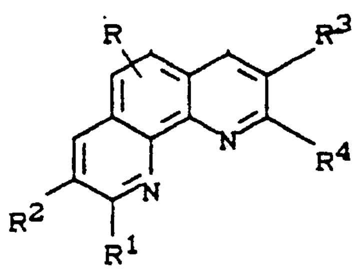

(I)

in welcher
R, $R^1$, $R^2$, $R^3$ und $R^4$ die in der Beschreibung angegebene Bedeutung haben, ihre Säureadditionssalze und Metallsalzkomplexe und ihre Verwendung zur Bekämpfung von Schädlingen, insbesondere von Pilzen; weiterhin die Ausgangsverbindungen, die 8-Amino- bzw. 8-Nitrochinoline.

Die neuen Hetaryl-1,10-phenanthrolin-Derivate sind durch die Formel (I) allgemein definiert und können hergestellt werden, z.B. aus geeigneten 8-Aminochinolinen und geeigneten Aldehyden. Die ebenfalls neuen 8-Aminochino line sind durch die Formel (II) allgemein definiert und können aus geeigneten 8-Nitrochinolinen mit Katalysatoren erhalten werden, wobei die ebenfalls neuen 8-Nitro-chinoline aus 8-Nitro-halogenchinolinen mit geeigneten Aminen hergestellt werden.

EP 0 334 148 A2

## Hetaryl-1,10-phenanthrolin-Derivate

Die vorliegende Erfindung betrifft neue Hetaryl-1,10-phenanthrolin-Derivate, mehrere Verfahren zu ihrer Herstellung sowie ihre Verwendung in Schädlingsbekämpfungsmitteln, vor allem als Fungizide.

Es ist bereits bekannt geworden, daß bestimmte Tetrahydrophthalimide, wie beispielsweise cis-N-[-(Trichlormethyl)thio]-4-cyclohexen-1,2-dicarboimid, fungizide Eigenschaften aufweisen (vgl. z.B. Science, (Washington) 115, 84 (1952); US 2 553 770).

Die Wirkung dieser Verbindungen ist jedoch in bestimmten Indikationsbereichen, insbesondere bei niedrigen Aufwandmengen und -konzentrationen, nicht immer ganz befriedigend.

Es wurden neue Hetaryl-1,10-phenanthrolin-Derivate der Formel (1)

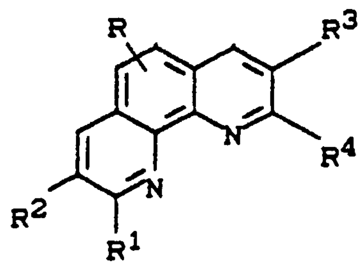

(I)

gefunden,
in welcher
$R^1$ und $R^4$ gleich oder verschieden sind und für Wasserstoff oder Alkyl stehen,
$R^2$ und $R^3$ gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedrigen Ring, der Sauerstoff, Schwefel und/oder Stickstoff als weitere Heteroatome enthalten kann, stehen,
sowie deren Säureadditions-Salze und Metallsalz-Komplexe.

Weiterhin wurde gefunden, daß man die neuen Hetaryl-1,10-phenanthrolin-Derviate der Formel (I)

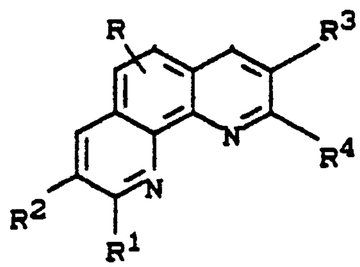

(I)

in welcher
$R^1$ und $R^4$ gleich oder verschieden sind und für Wasserstoff oder Alkyl stehen,
$R^2$ und $R^3$ gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedrigen Ring, der Sauerstoff, Schwefel und/oder Stickstoff als weitere Heteroatome enthalten kann, stehen,
bzw. deren Säureadditions-Salze und Metallsalz-Komplexe erhält, wenn man 8-Aminochinoline der Formel (II)

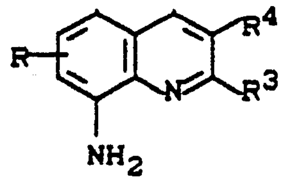

(II)

in welcher
$R^3$, $R^4$ und R die oben angegebene Bedeutung haben,
α) mit Aldehyden der Formel (III)

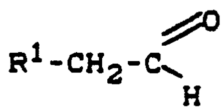

(III)

in welcher
$R^1$ die oben angegebene Bedeutung hat,
gegebenenfalls in Gegenwart von Verdünnungsmitteln und gegebenenfalls in Gegenwart starker Säuren cyclisiert und die so erhaltenen Produkte gegebenenfalls in Säureadditions-Salze oder Metallsalz-Komplexe überführt, oder
β) mit α,β-ungesättigten Aldehyden der Formel (IIIa)

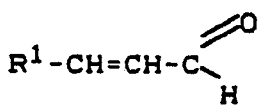

(IIIa)

in welcher
$R^1$ die oben angegebene Bedeutung hat,
gegebenenfalls in Gegenwart von Verdünnungsmitteln und gegebenenfalls in Gegenwart einer starken Säure oder gegebenenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze oder Metallsalz-Komplexe überführt.

Außerdem wurde gefunden, daß sich die neuen Hetaryl-1,10-phenanthrolin-Derivate der Formel (I) und deren Säureadditions-Salze und Metallsalz-Komplexe durch starke biologische Eigenschaften auszeichnen.

Überraschenderweise zeigen die erfindungsgemäßen Hetaryl-1,10-phenanthrolin-Derivate der Formel (I) sowie deren Säureadditions-Salze und Metallsalz-Komplexe eine bessere Wirkung gegen Schädlinge, insbesondere Pilze, als das aus dem Stand der Technik bekannte cis-N-((Trichlormethyl)thio)-4-cyclohexen-1,2-dicarboimid.

Die erfindungsgemäßen Hetaryl-1,10-phenanthrolin-Derivate stellen somit eine wertvolle Bereicherung der Technik dar.

Die erfindungsgemäßen Hetaryl-1,10-phenanthrolin-Derivate sind durch die Formel (I) allgemein definiert. Bevorzugt werden diejenigen Verbindungen der Formel (I),
in welcher
$R^1$ und $R^4$ gleich oder verschieden sind und für Wasserstoff oder geradkettiges oder verzweigtes Alkyl mit 1 bis 6 Kohlenstoffatomen stehen,
$R^2$ und $R^3$ gleich oder verschieden sind und für Wasserstoff oder geradkettiges oder verzweigtes Alkyl mit 1 bis 6 Kohlenstoffatomen stehen und
R für die Gruppe $-NR^5R^6$ steht,
in welcher
$R^5$ und $R^6$ gemeinsam mit dem Stickstoffatom, an welches sie gebunden sind, für einen gegebenenfalls ein- bis mehrfach, gleich oder verschieden durch $C_1$-$C_4$-Alkyl substituierten 3- bis 7-gliedrigen Ring, der gleiche oder verschiedene, ein oder mehrere Heteroatome wie Sauerstoff, Schwefel und/oder Stickstoff enthalten kann, stehen.

Besonders bevorzugt werden diejenigen Verbindungen der Formel (I), in welchen
$R^1$ und $R^4$ gleich oder verschieden sind und für Wasserstoff, Methyl, Ethyl, n- oder i-Propyl, n-, i-, s- oder t-

Butyl stehen,
$R^2$ und $R^3$ gleich oder verschieden sind und für Wasserstoff, Methyl, Ethyl, n- oder i-Propyl, n-, i-, s- oder t-Butyl stehen und
R für Aziridinyl, Azetidinyl, Pyrrolidinyl, Piperidinyl, Azepanyl, Piperazinyl, N-Methyl-piperazinyl, N-Ethyl-piperazinyl, N-Propyl-piperazinyl, 4-Methyl-pyrazinyl, Morpholinyl, Thiomorpholinyl, 1,4-Thiazinyl, 1,4-Oxazinyl, Pyrrolyl, Pyrazolyl, Imidazolyl oder Triazolyl steht.

Erfindungsgemäße Verbindungen sind auch Additionsprodukte aus Säuren und denjenigen Hetaryl-1,10-phenanthrolin-Derivaten der Formel (I) in denen $R^1$, $R^2$, $R^3$, $R^4$ und R diejenigen Bedeutungen haben, die im Zusammenhang mit der Beschreibung der erfindungsgemäßen Stoffe bereits für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach bis mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide, wie z.B. Phthalimid, Saccharin und Thiosaccharin.

Außerdem bevorzugte erfindungsgemäße Verbindungen sind auch Additionsprodukte aus Salzen von Metallen der I., II. und III. Hauptgruppe sowie des Zinns, sowie ferner Salze von Metallen der I., II., VII. und VIII. Neben gruppe des Periodensystems der Elemente und denjenigen Hetaryl-1,10-Phenanthrolin-Derivaten der Formel (I), in denen $R^1$, $R^2$, $R^3$, $R^4$ und R die Bedeutungen haben, die bereits im Zusammenhang mit der Beschreibung der erfindungsgemäßen Stoffe der Formel (I) für diese Substituenten genannt wurden.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Hetaryl-1,10-phenanthrolin-Derivate der allgemeinen Formel (I) genannt:

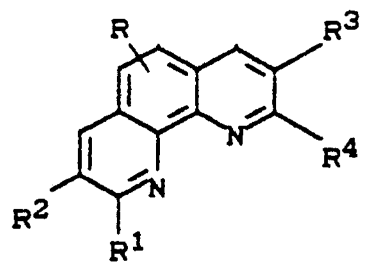

(I)

T a b e l l e: 1

| $R^1$ | $R^2$ | $R^3$ | $R^4$ | R |
|---|---|---|---|---|
| $CH_3$ | H | $CH_3$ | $C_2H_5$ | 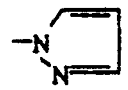 |
| $CH_3$ | H | H | $CH_3$ | 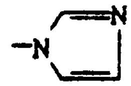 |
| $CH_3$ | H | $C_2H_5$ | $n-C_3H_7$ | 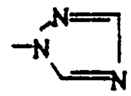 |
| $C_2H_5$ | $CH_3$ | $CH_3$ | $C_2H_5$ | 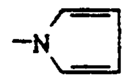 |
| $C_2H_5$ | $CH_3$ | H | $CH_3$ | 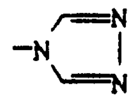 |
| H | H | $CH_3$ | $C_2H_5$ | 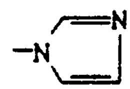 |
| H | H | H | $CH_3$ | 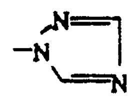 |
| H | H | $n-C_3H_7$ | $n-C_4H_9$ | 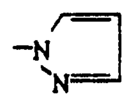 |
| $C_2H_5$ | $CH_3$ | H | H | 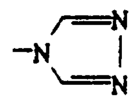 |
| $CH_3$ | H | $CH_3$ | $CH_3$ | 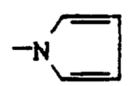 |
| $CH_3$ | H | $C_2H_5$ | H | 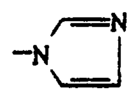 |
| $C_2H_5$ | $CH_3$ | $CH_3$ | $CH_3$ | 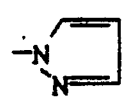 |
| $C_2H_5$ | $CH_3$ | $C_2H_5$ | H | 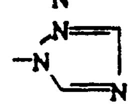 |
| $CH_3$ | H | $C_2H_5$ | $CH_3$ | 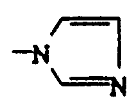 |
| $C_3H_7-n$ | $C_2H_5$ | H | $CH_3$ | 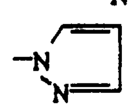 |

**Tabelle: 1 - Fortsetzung**

| $R^1$ | $R^2$ | $R^3$ | $R^4$ | R |
|---|---|---|---|---|
| $CH_3$ | H | H | $C_4H_9$-n | 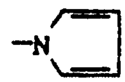 |
| $CH_3$ | H | $CH_3$ | $C_2H_5$ | 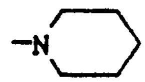 |
| $CH_3$ | H | H | $CH_3$ | 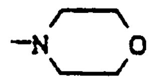 |
| $CH_3$ | H | $C_2H_5$ | $C_3H_7$-n | 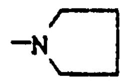 |
| $C_2H_5$ | $CH_3$ | H | $CH_3$ | 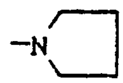 |
| H | H | $CH_3$ | $C_2H_5$ | 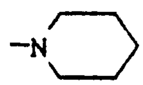 |
| H | H | H | $CH_3$ | 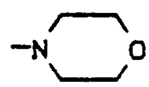 |
| H | H | $C_3H_7$-n | $C_4H_9$-n | 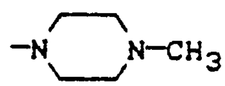 |
| $C_2H_5$ | $CH_3$ | H | H | 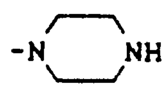 |
| $CH_3$ | H | $CH_3$ | $CH_3$ | 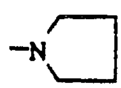 |
| $CH_3$ | H | $C_2H_5$ | H | 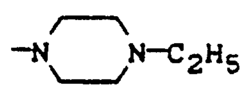 |
| $C_2H_5$ | $CH_3$ | $CH_3$ | $CH_3$ | 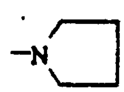 |
| $CH_3$ | H | $C_2H_5$ | $CH_3$ | 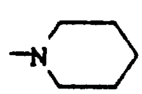 |

T a b e l l e : 1 - Fortsetzung

| $R^1$ | $R^2$ | $R^3$ | $R^4$ | R |
|---|---|---|---|---|
| $C_3H_7$-n | $C_2H_5$ | H | $CH_3$ | 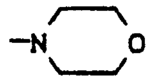 |
| $C_2H_5$ | $CH_3$ | $C_2H_5$ | H | 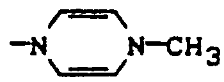 |
| $CH_3$ | H | H | $C_4H_9$-n | 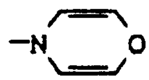 |
| $CH_3$ | H | $CH_3$ | $CH_3$ | 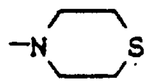 |
| $C_2H_5$ | $CH_3$ | H | H | 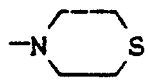 |
| H | H | $C_3H_7$-n | $C_4H_9$-n | 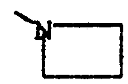 |
| H | H | H | $CH_3$ | 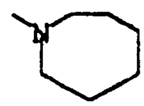 |
| H | H | $CH_3$ | $C_2H_5$ | 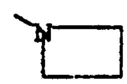 |
| $C_2H_5$ | $CH_3$ | H | $CH_3$ | 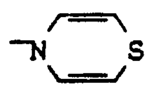 |
| $CH_3$ | H | $C_2H_5$ | $C_3H_7$-n | 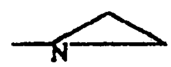 |
| $CH_3$ | H | H | $CH_3$ | 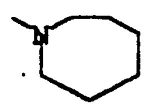 |
| $CH_3$ | H | $CH_3$ | $C_2H_5$ | 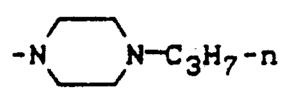 |
| $CH_3$ | H | $CH_3$ | $C_2H_5$ | 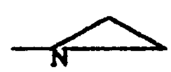 |

Verwendet man als Ausgangsstoffe beispielsweise 8-Amino-2-methyl-5-pyrrolidino-chinolin und Propionaldehyd, so läßt sich der Reaktionsablauf des Herstellungsverfahrens (α) durch das folgende Formelschema darstellen:

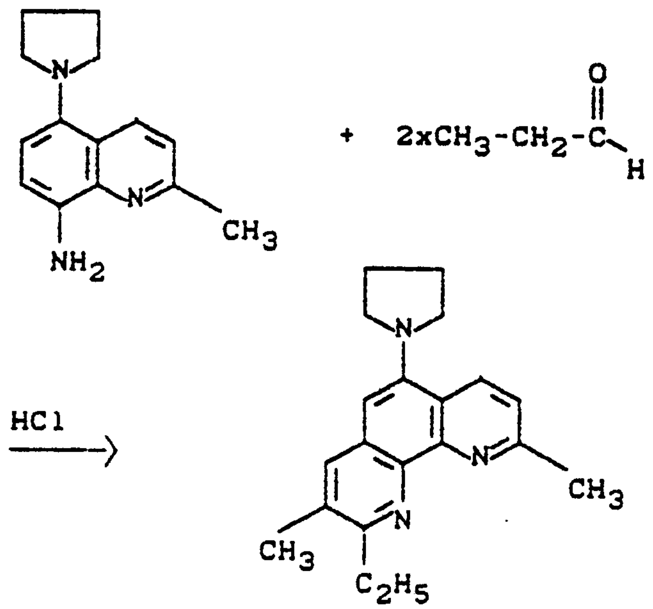


Verwendet man als Ausgangsstoffe beispielsweise 8-Amino-5-imidazolyl-2-methyl-chinolin und Crotonaldehyd, so läßt sich der Reaktionsablauf des Herstellungsverfahrens (β) durch das folgende Formelschema darstellen:

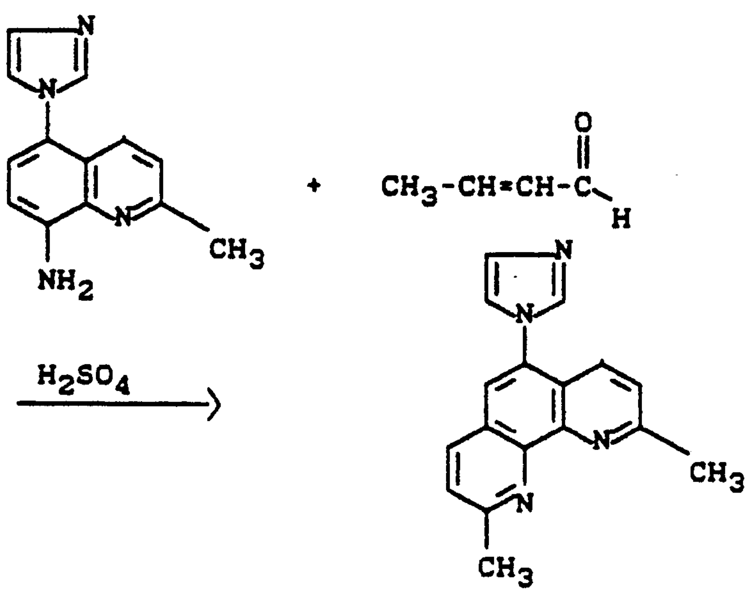


Die zur Durchführung der erfindungsgemäßen Verfahrensvarianten α und β als Ausgangsstoffe benötigten 8-Aminochinoline sind durch die Formel (II) allgemein definiert. In dieser Formel (II) stehen R, $R^3$ und $R^4$ vorzugsweise bzw. insbesondere für diejenigen Substituenten, die oben bei der Beschreibung der neuen Hetaryl-1,10-phenanthrolin-Derivate der Formel (I) als bevorzugt bzw. besonders bevorzugt für diese Reste genannt wurden.

Die 8-Aminochinoline der Formel (II) sind neu. Man erhält die Verbindungen der Formel (II), indem man 8-Nitro-chinoline der Formel (IV)

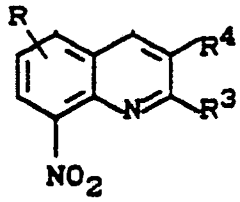

(IV)

in welcher

R, $R^3$ und $R^4$ die oben angegebene Bedeutung haben,

gegebenenfalls in Gegenwart eines Verdünnungsmittels in üblicher Art und Weise in Gegenwart eines Katalysators, wie beispielsweise Raney-Nickel oder mit Zinn-II-Salzen bei Temperaturen zwischen 20° C und 150° C und gegebenenfalls unter Druck zwischen 2 und 100 bar reduziert.

Als Lösungsmittel zur Herstellung der Verbindungen der Formel (II) kommen wäßrige Systeme, anorganische Säuren oder inerte organische Lösungsmittel infrage. Vorzugsweise verwendet man Wasser, Salzsäure oder aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldiemthyl oder -diethylether; Ketone, wie Aceton oder Butanon; Nitrile, wie Acetonitril oder Propionitril; Amide, wie Diemthylformamid, Dimethylacetamid, N-Methylformanilid, N-Methylpyrrolidon oder Hexamethylphosphortriamid; Ester, wie Essigsäureethylester oder Sulfoxide, wie Dimethylsulfoxid.

Die Reaktionstemperaturen können bei der Herstellung der 8-Thinochinoline der Formel (II) in einem größeren Bereich variiert werden. Im allgemeinen arbeitet man bei Temperaturen zwischen 20° C und 150° C, vorzugsweise bei Temperaturen zwischen 70° C und 100° C.

Als Reduktionsmittel zur Herstellung der 8-Aminochinoline der Formel (II) kommen alle üblicherweise für derartige Reduktionen verwendbaren Reduktionsmittel infrage. Vorzugsweise verwendet man Zinn-(II)-Salze oder Wasserstoff/Raney Nickel.

Zur Herstellung der 8-Aminochinoline der Formel (II) setzt man pro Mol 8-Nitrochinolin der Formel (IV) im allgemeinen 2,0 bis 6,0 Mol, vorzugsweise 4,0 bis 5,0 Mol Reduktionsmittel ein.

Die Herstellung der 8-Aminochinoline kann im allgemeinen bei Normaldruck durchgeführt weden. Unter bestimmten Voraussetzungen kann jedoch auch unter erhöhtem Druck gearbeitet werden.

Die Reaktionsführung, Aufarbeitung und Isolierung der Reaktionsprodukte der Formel (II) erfolgt in allgemein üblicher Art and Weise.

Die zur Herstellung der 8-Aminochinoline als Ausgangsstoffe benötigten 8-Nitrochinoline sind durch die Formel (IV) allgemein definiert. In dieser Formel (IV) stehen R, $R^3$ und $R^4$ vorzugsweise bzw. insbesondere für diejenigen Substituenten, die oben bei der Beschreibung der neuen Hetaryl-1,10-phenanthrolin-Derivate der Formel (I) als bevorzugt bzw. besonders bevorzugt für diese Reste genannt wurden.

Die 8-Nitrochinoline der Formel (IV) sind neu. Man erhält die Verbindungen der Formel (IV), indem man 8-Nitro-halogenchinoline der Formel (V)

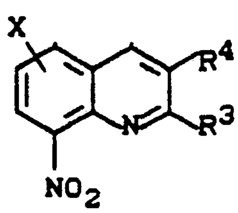

(V)

in welcher

$R^3$ und $R^4$ die oben angegebene Bedeutung haben und

X für Halogen, besonders für Chlor und Brom, insbesondere Chlor steht

mit Aminen der Formel (VI)

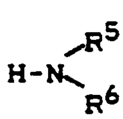

(VI)

in welcher
$R^5$ und $R^6$ die oben angegebene Bedeutung haben
gegebenenfalls in Gegenwart eines Verdünnungsmittels und gegebenenfalls in Gegenwart eines Säureakzeptors umsetzt.

Die 8-Nitro-halogenchinoline der Formel (V) sind bekannt und/oder können nach bekannten Verfahren in einfacher, analoger Weise hergestellt werden (vgl. z.B. Org. Reactions 7, 59ff., (1953); Kirk-Othmer, 2.Auflage, 15, 869 und DE-OS 37 09 263).

Die Amine der Formel (VI) sind allgemein bekannte Verbindungen der organischen Chemie.

Als Verdünnungsmittel zur Herstellung der neuen 8-Nitro-chinoline der Formel (IV) kommen alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Dimethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethyl formamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem Verfahren zur Herstellung der neuen 8-Nitrochinoline der Formel (IV) in einem größeren Bereich variiert werden. Im allgemeinen arbeitet man bei Temperaturen zwischen 20° C und 250° C, vorzugsweise bei Temperaturen zwischen 80° C und 150° C.

Zur Durchführung des Verfahrens zur Herstellung der neuen 8-Nitrochinoline der Formel (IV) setzt man pro Mol 8-Nitrohalogenchinolin der Formel (V) 1,0 bis 3,5 Mol, vorzugsweise 1,0 bis 1,5 Mol an Aminen der Formel (VI) ein.

Die Reaktionen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt. Die Aufarbeitung erfolgt nach üblichen Methoden.

Als Säureakzeptoren können bei dem Verfahren zur Herstellung der neuen Verbindungen der Formel (IV) alle üblicherweise für derartige Umsetzungen verwendbaren Säurebindemittel eingesetzt werden. Vorzugsweise infrage kommen Alkalimetallhydroxide wie z. B. Natrium- und Kaliumhydroxid, Erdalkalihydroxide wie z. B. Calcium hydroxid, Alkalicarbonate und - alkoholate wie Natrium-und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die außerdem als Ausgangsstoffe zur Herstellung der erfindungsgemäßen Verbindungen der Formel (I) zu verwendenden Aldehyde sind durch die Formeln (III) und (IIIa) allgemein definiert. In den Formeln (III) und (IIIa) hat der Rest $R^1$ vorzugsweise bzw. insbesondere diejenige Bedeutung, die oben im Zusammenhang mit der Beschreibung der erfindungsgemäßen Stoffe der Formel (I) als bevorzugt angegeben ist.

Die Verbindungen der Formeln (III) und (IIIa) sind allgemein bekannte Verbindungen der organischen Chemie.

Als Verdünnungsmittel zur Durchführung des Herstellungsverfahrens (α) und (β) kommen Mischungen von starken anorganischen oder organischen Säuren mit Wasser oder inerten organischen Lösungsmitteln infrage.

Säuren, welche zur Durchführung des Herstellungsverfahrens (α) oder (β) verwendet werden können sind beispielsweise Schwefelsäure, Salzsäure und p-Toluolsulfonsäure.

Zur Durchführung des Herstellungsverfahrens (α) oder (β) können praktisch alle inerten organischen Lösungsmittel verwendet werden, insbesondere aromatische, gegebenenfalls halogenierte Kohlenwasserstoffe, wie beispielsweise, Benzin, Benzol, Toluol, Xylol, Chlorbenzol, Dichlorbenzol, Petrolether, Pentan, Hexan, Heptan, Ligroin oder Cyclohexan.

Die Reaktionstemperaturen können bei der Durchführung der Herstellungsverfahren (α) und (β) in einem größeren Bereich variiert werden. Im allgemeinen arbeitet man bei Temperaturen zwischen 20 °C und 180 °C, vorzugsweise bei Temperaturen zwischen 50 °C und 130 °C.

Als Oxidationsmittel zur Durchführung des Herstellungsverfahrens (β) kommen die für derartige Reaktionen üblichen Oxidationsmittel infrage; vorzugsweise verwendet man Arsensäure oder Nitrogruppen enthaltende aromatische Säuren oder deren Alkalimetall-Salze, wie beispielsweise 3-Nitrobenzolsulfonsäure.

Zur Durchführung der Herstellungsverfahren (α) und (β) setzt man pro Mol 8-Aminochinolin der Formel (II) 1,0 bis 4,0 Mol, vorzugsweise, 1,5 bis 3,5 Mol an Aldehyden der Formel (III) oder (IIIa) und gegebenenfalls 1,0 bis 2,0 Mol, vorzugsweise 1,0 Mol Oxidationsmittel ein. Die Reaktionsführung, Aufarbeitung und Isolierung der Reaktionsprodukte der Formel(I) erfolgt in allgemein üblicher Art und Weise.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metall salz-Komplexe in bekannter Weise, z.B. durch Abfiltrieren, isolieren und gegebenenfalls durch Umkristallisation reinigen.

Die erfindungsgemäßen Wirkstoffe weisen ein starke biologische Wirkung auf und können zur Bekämpfung von unerwünschten Schädlingen praktisch eingesetzt werden. Die Wirkstoffe sind z. B. für den Gebrauch als Pflanzenschutzmittel einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg protektiv zur Bekämpfung von Venturia-Arten bei Äpfeln eingesetzt werden.

Außerdem besitzen die erfindungsgemäßen Wirkstoffe eine gute fungizide Wirkung gegen Pyricularia-Arten an Reis sowie gegen Erysiphe graminis und Leptosphaeria nodorum an Getreide.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermittel und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohol, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillinit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxy ethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es Kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und die Verwendung der erfindungsgemäßen Wirkstoffe geht aus den folgenden Beispielen hervor.

<u>Herstellungsbeispiele</u>

<u>Beispiel 1:</u>

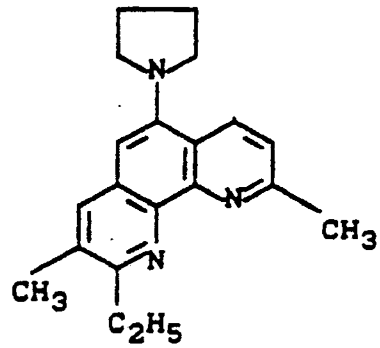

(I-1)

(Verfahren α)

11,4 g (0.05 Mol) 8-Amino-2-methyl-5-pyrrolidinochinolin und 6,7 g (0.05 Mol) wasserfreies Aluminiumchlorid werden in 50 ml Methylenchlorid vorgelegt und 30 Minuten bei Rückflußtemperatur erhitzt. Nach dem Abkühlen auf Raumtemperatur werden 5,8 g (0,10 Mol) Propionaldehyd in 20 ml Methylenchlorid zugetropft und das Gemisch 4 Stunden am Rückfluß erhitzt. Der nach dem Einengen verbleibende Rückstand wird mit Wasser versetzt und mehrere Male mit Methylenchlorid extrahiert. Die organische Phase wird unter vermindertem Druck eingeengt und über eine Kieselgelsäule (Laufmittel: Methylenchlorid) chromatographiert.

Man erhält 5,1 g (32 % der Theorie) 2-Ethyl-3,9-dimethyl-6-pyrrolidino-1,10-phenanthrolin vom Schmelzpunkt 106-110° C.

Beispiel 2:

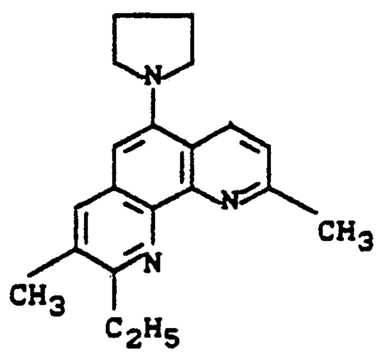

(I-2)

x $ZnCl_2$ x 1/2 $H_2O$

(Verfahren α, 2.Stufe, Salzbildung)

Zu 2,1 g (0,007 Mol) des nach Beispiel 1 erhaltenen 2-Ethyl-3,9-dimethyl-6-pyrrolidino-1,10-phenanthrolin in 30 ml wasserfreiem Ethanol werden bei Raumtemperatur 1,0 g (0,007 Mol) Zinkchlorid in 30 ml wasserfreiem Ethanol zugegeben und das Gemisch 5 Minuten unter Rückfluß zum Sieden erhitzt. Nach dem Abkühlen auf Raumtemperatur wird der Niederschlag abgesaugt, mit Ethanol und mit Wasser gewaschen und getrocknet.

Man erhält 2,6 g (84 % der Theorie) des $ZnCl_2$-Komplexes des 2-Ethyl-3,9-dimethyl-6-pyrrolidino-1,10-phenanthrolins mit einem Schmelzpunkt von 311-313° C.

Beispiel 3:

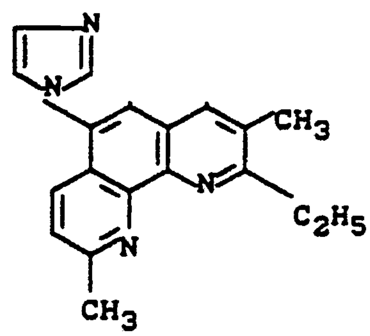

(I-3)

In analoger Weise zu den in den Beispielen 1 und 2 beschriebenen Methoden und unter Berücksichtigung der Angaben in den Beschreibungen zu den erfindungsgemäßen Verfahren wird 2-Ethyl-3,9-dimethyl-6-i imidazol-1-yl-1,10-phenanthrolin erhalten.
($^1$H-NMR δ = 2,6 (s, 3H); 2,95 ) (s, 3H)*.
(* Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.)

Herstellung der Ausgangsstoffe

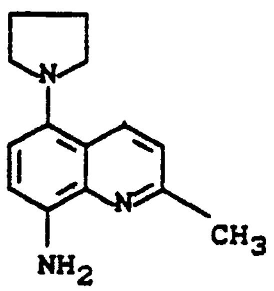

(II-1)

Zu 60,1 g (0,266 Mol) Zinn(II)-chlorid-dihydrat in 200 ml konzentrierter Salzsäure werden 22,8 g (0,089 Mol) 2-Methyl-8-nitro-5-pyrrolidino-chinolin in 100 ml konzentrierter Salzsäure zugetropft, wobei die Temperatur auf 50° C ansteigt. Nach erfolgter Zugabe wird eine Stunde bei 70° C weiter erhitzt. Danach wird die Reaktionsmischung abgekühlt, auf Eis gegossen, mit Natronlauge alkalisch gestellt und mehrmals mit Methylenchlorid extrahiert. Die vereinigten organischen Phasen werden gewaschen, mit Natriumsulfat getrocknet und eingeengt.
Man erhält 18,1 g (90 % der Theorie) 8-Amino-2-methyl-5-pyrrolidino-chinolin als Öl.

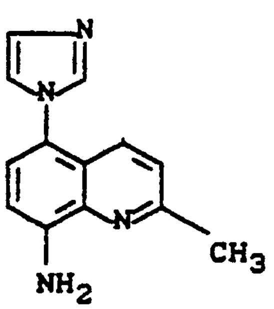

(II-2)

In analoger Weise zu der im Beispiel (II-1) beschriebenen Methode und unter Berücksichtigung der Angaben in den Beschreibungen zu dem erfindungsgemäßen Verfahren wird 8-Amino-2-methyl-5-imidazol-1-yl-chinolin in einer Ausbeute von 81 % der Theorie mit dem Schmelzpunkt 166-168° C erhalten.

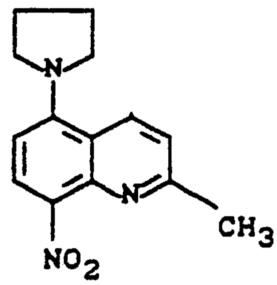

(IV-1)

28 g (0,126 Mol) 5-Chlor-2-methyl-8-nitro-chinolin und 25 g (0,352 Mol) Pyrrolidin werden in 150 ml wasserfreiem Toluol 8 Stunden bei 80° C gerührt. Nach dem Abkühlen auf Raumtemperatur wird mit Wasser chloridfrei gewaschen, die organische Phase getrocknet und unter vermindertem Druck das Lösungsmittel abdestilliert. Man erhält 24,4 g (75 % der Theorie) 2-Methyl-8-nitro-5-pyrrolidino-chinolin vom Schmelzpunkt 144-145° C.

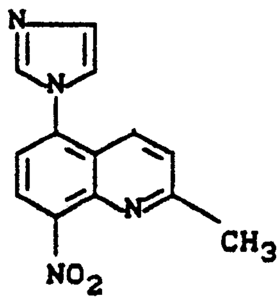

(IV-2)

28 g (0,126 Mol) 5-Chlor-2-methyl-8-nitro-chinolin und 25,7 g (0,378 Mol) Imidazol werden 8 Stunden bei 150° C geschmolzen. Nach dem Abkühlen auf Raumtemperatur wird die Schmelze mit Wasser versetzt und kräftig gerührt. Nach dem Absaugen wird der Rückstand mit Methanol gewaschen und getrocknet. Man erhält 21,1 g (65 % der Theorie) 5-(Imidazol-1-yl)-2-methyl-8-nitro-chinolin vom Schmelzpunkt 208-212° C.

## Anwendungsbeispiel

In dem folgenden Anwendungsbeispiel wird die nachstehend aufgeführte Verbindung als Vergleichssubstanz eingesetzt:

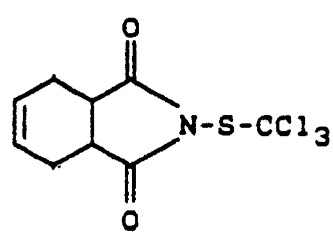

(A)

cis-N-((Trichlormethyl)thio)-4-cyclohexen-1,2-dicarboimid

(bekannt aus Science, (Washington) 115, 84 (1952); US-PS 2 553 770).

Beispiel A

Venturia-Test (Apfel) /protektiv

Lösungsmittel: 4.7 Gewichtsteile Aceton
Emulgator: 0.3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigt z.B. der erfindungsgemäße Stoff [(I)-1] eine bessere Wirksamkeit als die Vergleichssubstanz (A).

**Ansprüche**

1. Hetaryl-1,10-phenanthrolin-Derivate der Formel (I)

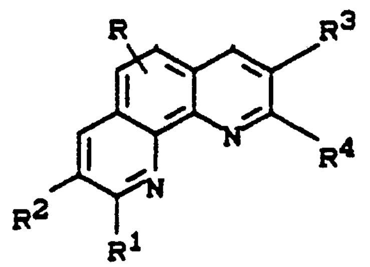

(I)

in welcher
$R^1$ und $R^4$ gleich oder verschieden sind und für Wasserstoff oder Alkyl stehen,
$R^2$ und $R^3$ gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedrigen Ring, der weitere, gleiche oder verschiedene Heteroatome enthalten kann, stehen
sowie deren Säureadditionssalze und Metallsalzkomplexe.

2. Hetaryl-1,10-phenanthrolin-Derivate gemäß Anspruch 1, wobei in der Formel (I)
$R^1$ und $R^4$ gleich oder verschieden sind und für Wasserstoff oder geradkettiges oder verzweigtes Alkyl mit 1 bis 6 Kohlenstoffatomen stehen,
$R^2$ und $R^3$ gleich oder verschieden sind und für Wasserstoff oder geradkettiges oder verzweigtes Alkyl mit 1 bis 6 Kohlenstoffatomen stehen und
R für die Gruppe $-NR^5R^6$ steht,
in welcher
$R^5$ und $R^6$ gemeinsam mit dem Stickstoffatom, an welches sie gebunden sind, für einen gegebenenfalls ein- bis mehrfach, gleich oder verschieden durch $C_1$-$C_4$-Alkyl substituierten 3- bis 7-gliedrigen Ring, der gleiche oder verschiedene, ein oder mehrere Sauerstoff-, Schwefel und/oder Stickstoffatome enthalten kann, sowie deren Säureadditionssalze und Metallsalz-Komplexe.

3. Hetaryl-1,10-phenanthrolin-Derivate gemäß Anspruch 1, wobei in der Formel (I)
$R^1$ und $R^4$ gleich oder verschieden sind und für Wasserstoff, Methyl, Ethyl, n- oder i-Propyl, n-, i-, s- oder t-Butyl stehen,

$R^2$ und $R^3$ gleich oder verschieden sind und für Wasserstoff, Methyl, Ethyl, n- oder i-Propyl, n-, i-, s- oder t-Butyl stehen und
R für Aziridinyl, Azetidinyl, Pyrrolidinyl, Piperidinyl, Azepanyl, Piperazinyl, N-Methylpiperazinyl, N-Ethyl-piperazinyl, N-Propyl-piperazinyl, 4-Methyl-pyrazinyl, Morpholinyl, Thiomorpholinyl, 1,4-Thiazinyl, 1,4-Oxazinyl, Pyrrolyl, Pyrazolyl, Imidazolyl oder Triazolyl steht,
sowie die Säureadditionssalze und Metallsalz-Komplexe.

4. Verfahren zur Herstellung von Hetaryl-1,10-phenanthrolin-Derivaten der Formel (I)

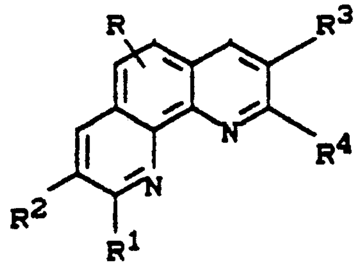

(I)

in welcher
$R^1$ und $R^4$ gleich oder verschieden sind und für Wasserstoff oder Alkyl stehen,
$R^2$ und $R^3$ gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedrigen Ring, der weitere, gleiche oder verschiedene Heteroatome enthalten kann, stehen
sowie deren Säureadditions-Salze und Metallsalz-Komplexe, dadurch gekennzeichnet, daß man 8-Aminochinoline der Formel (II)

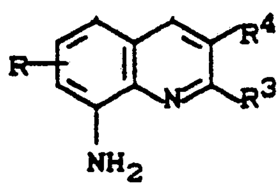

(II)

in welcher
$R^3$, $R^4$ und R die oben angegebene Bedeutung haben,
α) mit Aldehyden der Formel (III)

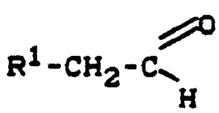

(III)

in welcher
$R^1$ die oben angegebene Bedeutung hat,
gegebenenfalls in Gegenwart von Verdünnungsmitteln und gegebenenfalls in Gegenwart starker Säuren cyclisiert und die so erhaltenen Produkte gegebenenfalls in Säureadditions-Salze oder Metallsalz-Komplexe überführt, oder
β) mit α,β-ungesättigten Aldehyden der Formel (IIIa)

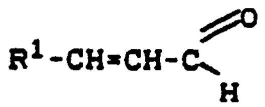

(IIIa)

in welcher
R' die oben angegebene Bedeutung hat,
gegebenenfalls in Gegenwart von Verdünnungsmitteln und gegebenenfalls in Gegenwart einer starken Säure oder gegebenenfalls in Gegenwart eines Oxidationsmittels oder unter Wasserabscheidung gegebenenfalls in Gegenwart eines Katalysators cyclisiert und die so erhaltenen Produkte gegebenenfalls in Säureadditions-Salze oder Metallsalz-Komplexe überführt.

5. Schädlingsbekämpfungsmittel, gekennzeichnet durch einen Gehalt an mindestens einem Hetaryl-1,10-phenanthrolin-Derivat der Formel (I) nach den Ansprüchen 1 und 4.

6. Verwendung von Hetaryl-1,10-phenanthrolin-Derivaten der Formel (I) nach den Ansprüchen 1 und 4 zur Bekämpfung von Schädlingen.

7. Verfahren zur Bekämpfung von Schädlingen, dadurch gekennzeichnet, daß man Hetaryl-1,10-phenanthrolin-Derivate der Formel (I) nach den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man Hetaryl-1,10-phenanthrolin-in-Derivate der Formel (I) nach den Ansprüchen 1 und 4 mit Streckmitteln und/oder oberflächenaktiven Mitteln vermischt.

9. 8-Aminochinoline der Formel (II)

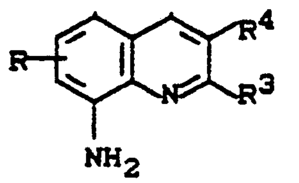

(II)

in welcher
$R^3$ und $R^4$ gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedrigen Ring, der weitere, gleiche oder verschiedene Heteroatome enthalten kann, stehen.

10. Verfahren zur Herstellung von 8-Aminochinolinen der Formel (II)

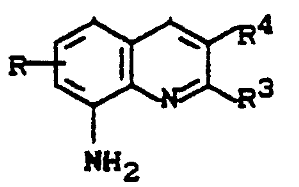

(II)

in welcher
$R^3$ und $R^4$ die gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedrigen Ring, der weitere, gleiche oder verschiedene Heteroatome enthalten kann, stehen,
dadurch gekennzeichnet, daß man 8-Nitro-chinoline der Formel

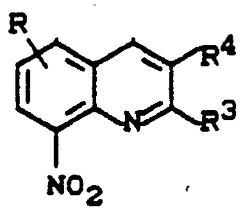

(IV)

in welcher
R, $R^3$ und $R^4$ die oben angegebene Bedeutung haben,
gegebenenfalls in Gegenwart eines Verdünnungsmittels und in Gegenwart eines Katalysators bei Temperaturen zwischen 20° C und 150° C und gegebenenfalls unter Druck zwischen 2 und 100 bar reduziert.

11. 8-Nitro-chinoline der Formel (IV)

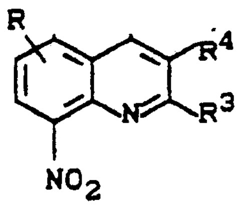

(IV)

in welcher
$R^3$ und $R^4$ gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedri gen Ring, der weitere, gleiche oder verschiedene Heteroatome enthalten kann, stehen.

12. Verfahren zur Herstellung von 8-Nitro-chinolinen der Formel (IV)

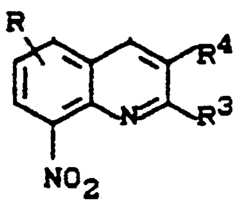

(IV)

in welcher
$R^3$ und $R^4$ gleich oder verschieden sind und für Wasserstoff oder Alkyl stehen und
R für die Gruppe $-NR^5R^6$ steht,
in welcher
$R^5$ und $R^6$ gemeinsam mit dem Stickstoffatom, an welches sie gebunden sind, für einen gegebenenfalls substituierten heterocyclischen 3- bis 7-gliedrigen Ring, der weitere, gleiche oder verschiedene Heteroatome enthalten kann, stehen,
dadurch gekennzeichnet, daß man 8-Nitro-halogenchinoline der Formel (V)

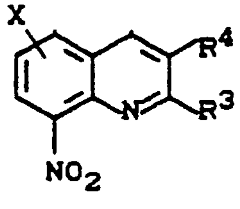

(V)

in welcher

$R^3$ und $R^4$ die oben angegebene Bedeutung haben und
X für Halogen steht
mit Aminen der Formel (VI)

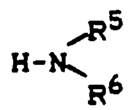

(VI)

in welcher
$R^5$ und $R^6$ die oben angegebene Bedeutung haben,
gegebenenfalls in Gegenwart eines Verdünnungsmittels und gegebenenfalls in Gegenwart eines Säureakzeptors umsetzt.